Europäisches Patentamt

European Patent Office

Office européen des brevets

(19)

(11) Numéro de publication: **0 073 173**
**B1**

(12) **FASCICULE DE BREVET EUROPEEN**

(45) Date de publication du fascicule du brevet: **30.05.90**

(21) Numéro de dépôt: **82401563.0**

(22) Date de dépôt: **20.08.82**

(51) Int. Cl.⁵: **C 07 G 17/00, A 23 L 1/30, A 61 K 7/48**

(54) Produits extraits d'animaux marins invertébrés, leur préparation et leurs utilisations pour usage pharmaceutique, en diététique et en cosmétologie.

(30) Priorité: **21.08.81 FR 8116127**

(43) Date de publication de la demande:
**02.03.83 Bulletin 83/09**

(45) Mention de la délivrance du brevet:
**30.05.90 Bulletin 90/22**

(84) Etats contractants désignés:
**CH DE FR GB LI NL**

(56) Documents cités:
**CH-A- 505 561**  **FR-A-2 508 768**
**DE-A-2 755 709**  **US-A-4 103 003**
**FR-A-1 272 933**  **US-A-4 208 405**
**FR-A-1 350 038**  **US-A-4 220 667**
**FR-A-2 108 027**

**CHEMICAL ABSTRACTS, vol. 50, no. 15, 10 Aou7t 1956, colonne 10879i-10880a,b,c, Columbus Ohio (USA);**

**CHEMICAL ABSTRACTS, vol. 40, no. 16, 20 Aou7t 1946, no. 4819(6), Columbus Ohio (USA);**

(73) Titulaire: **Lemonnier, Monique, née Perrier**
**"Les Bucoliques" 10 chemin aux Boeufs**
**F-14123 Ifs-Plaine (FR)**

(72) Inventeur: **Lemonnier, Monique, née Perrier**
**"Les Bucoliques" 10 chemin aux Boeufs**
**F-14123 Ifs-Plaine (FR)**

Courier Press, Leamington Spa, England.

(56) Documents cités:
CHEMICAL ABSTRACTS, vol. 39, no. 4, 20 févier
1945, colonne 732(9), Columbus Ohio (USA);

CHEMICAL ABSTRACTS, vol. 38, no. 20, 20
octobre 1944, colonne 5611(3), Columbus Ohio
(USA);

Grand Larousse Encyclopédique, vol. 4,
"Echinodermes"

Grande Larousse Encyclopédique, vol. 6,
"Invertébrés"

Encyclopedia Universalis, vol. 1, p. 1082
B.M.I.A. ZALENSKAYA et al., Comp. Biochem.
Physiol. vol. 65B, pp. 375-378

T. FARBU et al., Comp. Biochem. Physiol., vol.
63B, p. 395

L. CARIELLO et al., Comp. Biochem. Physiol.,
vol. 63B, pp. 7782

R.E. SCHEIBLING, Comp. Biochem. Physiol., vol.
67B, pp. 297-300

T. MATSUMURA, Comp. Biochem. Physiol., vol.
62B, pp. 101-105

Chemical Abstracts 73:128205v (1970)

Chemical Abstracts 82:71707q (1975)

**Description**

L'invention est relative à des produits, possédant notamment des propriétés reconstituantes et revitalisantes, à leur préparation et à leurs utilisations pour l'usage pharmaceutique, en diététique et en cosmétologie.

Pour remédier à certaines carences physiologiques entraînant le rachitisme, l'anémie, la fatigue, pour aider à la régénérescence et à la croissance, on a largement utilisé des produits obtenus à partir de poissons, tels que des autolysats de poisson ou encore de l'huile de foie de morue pour des carences internes, voire différents extraits à base d'oursins ou de lamellibranches pour des carences externes en cosmétologie.

On a pu remédier, tout au moins en partie, aux problèmes gustatifs posés par ces produits en les administrant sous des présentations solides ou par injections.

Ces produits, plus spécialement l'huile de foie de morue, présentant cependant l'inconvénient d'être plus ou moins bien tolérés par certains organismes. En particulier l'huile de foie de morue présente des teneurs élevées en graisses saturées d'origine animale, qui augmentent donc d'une manière désavantageuse le taux de choléstérol du patient.

Elle renferme également du squalène et du lanestérol dont l'assimilation peut être difficile.

La recherche de produits plus satisfaisants a conduit l'inventeur à étudier les composants obtebus à partir d'animaux marins autres que les poissons.

Il s'est avéré alors qu'en utilisant certains types d'animaux marins, il état possible de disposer de constituants précieux dont au moins le zinc révélé seulement par traces dans le Chemical Abstracts 73: 128 205 (1970) et le Chemical Abstracts 82:717 079 (1975), pour l'élaboration de produits de grande valeur possédant, en particulier vis-à-vis de l'organisme humain ou animal des propriétés reconstituantes ainsi que des propriétés revitali santes des tissus.

L'invention a donc pour but de fournir des produits posédant des propriétés reconstituantes et revitalisantes renfermant des composants obtenus à partir des éléments physiologiques d'animaux marins invertébrés, matière première largement répandue et par là, d'un grand intérêt économique.

Elle a pour but de fournir une préparation de mise en oeuvre aisée et dont le coût permet une exploitation à l'échelle industrielle.

Elle vis également à fournir des utilisations mettant à profit les propriétés reconstituantes et revitalisantes de l'invention pour l'usage pharmaceutique, la diététique humaine et animale et la cosmétologie.

Les produits de l'invention à propriétés reconstituantes et revitalisantes, facteurs d'équilibre métabolique et de croissance, sont caractérisés en ce qu'ils renferment au moins un composé du type de ceux extraits d'animaux marins invertébrés, de préférence des astéries ou étoiles de mer, de leurs différents éléments physiologiques tels que tissus, foie, estomac, gonades, sperme, ovaires, follicules, oeufs, glandes, sécrétions, organes, membranes, et liquides cellulaires de préférence.

D'une manière surprenante l'étude de cette classe d'animaux, en particulier des étoiles de mer, a montré qu'il était possible d'en extraire des composants utilisables chez l'homme et l'animal malgré la spécificité de ces composants vis-à-vis des espèces en question et les divers parasites rencontrés dans celles-ci qu'il est possible d'éliminer sélectivement. Connaissant la large distribution de par le monde de ces animaux on mesurera l'intérêt de leut utilisation comme matière première pour l'élaboration des produits de l'invention.

Selon un mode de réalisation de l'invention, les composants possédant des propriétés reconstituantes et revitalisantes en question entrant dans la composition des produits de l'invention sont des acides aminés et éventuellement des peptides et éventuellement des protéines et éventuellement des enzymes et éventuellement des hormones et éventuellement des vitamines et éventuellement des éléments minéraux, outre le zinc, seuls, sous forme de sels, complexes ou autres dérivés.

Parmi les acides aminés on citera le tryptophane: celui-ci étant détruit par l'hydrolyse chlorhydrique, on hydrolyse la solution protéique pendant 4 à 8 heures par du NaOH 2—4N ou hydrolyse enzymatique à température n'excédant pas 30°C.

Le mélange obtenu est fractionné et analysé par chromatographie sur résine échangeuse de cations selon la technique de MOORE et STEIN. Le tryptophane peut encore être caractérisé par la réaction d'ADAMKIEWIEZ-HOPKINS (caractéristique du noyau indole qui en milieu acide et à froid donne une coloration violette), ou l'on utilise une autre méthode usuelle.

On citera encore la lysine obtenue par la méthode de FOX, DUNN et STODDARD par exemple en se souvenant que la stérilisation l'altère et qu'elle peut être bloquée par la réaction de MAILLARD. Le procédé par synthèse de BUCHERER est fort connu. Enfin d'une façon générale on citera les acides aminés essentiels pour l'homme et les animaux, identifiés par la méthode HOWEVER.

D'autres composants sont constitués par des hormones tirées de différents organes tels que gonades, sperme, ovaires, follicules, oeufs, larves, tissus, sécrétions de préférence et éventuellement des enzymes telles que la pepsine extraite de la muqueuse stomacale puis préparée par exemple selon la procédé NORTHROP, et la trypsine (précurseurs pepsinogène, trypsinogène). L'isolation de la trypsine est obtenue de préférence par la méthode SALVATORE F. RUSSO et DEOBORAH J. YADOFF ou encore par celle de KUNITZ et NORTHROP par exemple; l'électrophorèse sur gel est réalisée aussi selon la méthode de PANYM et CHALKLEY de préférence, l'activité enzymatique est déterminée selon la méthode de HUMMEL et à la température de 35°C.

Selon un autre mode de réalisation de l'invention, ces composants sont constitués ou renferment en plus des constituants évoqués ci-dessus, des éléments minéraux, sous forme de sels, complexes ou autres dérivés, dont le zinc.

Un élément préféré est constitué par le fer. Lorsqu'il est employé sous forme de sels, les sels utilisés selon l'invention seront les sels ferreux et avantageusement l'oxalate $FeC_2O_42H_2O$ de fer dosé selon la posologie courant du Codex. On évitera les boissons au quinquina, cola, coca, rathantia, les vins médicinaux et autres prises dont l'antipyrine.

Parmi les autres éléments utilisés dans les produits de l'invention, seuls ou en combinaison, on citera, outre le zinc déjà cité, le calcium, le phosphore, le cuivre, le manganèse, le chrome, le fer, éléments minéraux sous forme de sels, complexes ou autres dérivés.

Le phosphore sera évité avec les sels ferreux. Il peut être déterminé par la méthode de BRIGGS.

Selon un autre mode préféré de l'invention, ces produites renferment en outre de la pectine. On utilise plus spécialement de la pectine sèche de pomme, de préférence.

D'une manière avantageuse, on note une interaction favorable de ces différents composants. La pectine présente ainsi l'avantage de diminuer en synergie avec le fer les fortes teneurs en cholestérol et de remédier à certains troubles de la digestion comme les calculs biliaires et les troubles intestinaux.

Grâce également à l'apport des enzymes digestives, protéolytiques par exemple contenues dans les protéines, extraites selon les méthodes usuelles à partir des étoiles de mer de préférence, et éventuellement des peptides, éventuellement des acides aminés, qui agissant notamment sur la pectine, la digestibilité et le transit se trouvent accrus. Comme déjà indiqué, l'étude des propriétés des composants obtenus à partir d'animaux marins invertébrés, de préférence des étoiles de mer, a montré qu'il était possible de disposer de composants de grande valeur quant à leurs propriétés reconstituantes vis-à-vis de l'organisme et même revitalisantes à l'égard des tissus, en particulier les protéines, éventuellement les peptides, éventuellement les enzymes, éventuellement les hormones, éventuellement les éléments minéraux, le zinc seul ou en combinaison et éventuellement les vitamines tirées des étoiles de mer de de préférence, extraits des différents éléments physiologiques tels que tissus, foie, estomac, gonades, sperme, ovaires, follicules, oeufs, glandes, sécrétions, organes, membranes et liquides cellulaires de préférence.

Les propriétés des produits de l'invention sont alors mises à profit pour l'élaboration de produits possèdant de remarquables qualités en tant que reconstituants et éventuellement revitalisants et utilisables de ce fait pour un usage pharmaceutique, en diététique et en cosmétologie.

L'invention vise donc également des produits pour usage pharmaceutique, des produits diététiques ou cosmétiques renfermant une quantité efficace d'au moins l'un des composants des produits évoqués ci-dessus.

Des compositions humaines préférées en diététique renferment par rapport au poids du produit final sec, 15 à 30% en poids de protéines, éventuellement de peptides, éventuellement d'acides aminés, avantageusement de l'ordre de 20 à 25% en poids, 0,15 à 0,50% en fer, de préférence 0,30 à 0,35%, et de 1 à 1,5%, plus spécialement de l'ordre de 1,3% de pectine. On peut incorporer des hormones et éventuellement des enzymes.

Les produits à usage pharmaceutique sont obtenus à partir de broyats ou ce sont des extraits.

Les produits renfermant la substance active appelée pectine, de préférence de la pectine sèche de pommes, sont obtenus à partir de produits extraits, broyats, extraits, obtenus à partir des éléments physiologiques d'animaux marins invertébrés.

L'utilisation des compositions de l'invention provoque le "coup de fouet" salutaire en limitant autant que faire se peut la teneur en cholestérol et an augmentant la tolèrance digestive ce qui est particulièrement appréciable dans le cas d'organismes délicats.

Les produits diététiques selon l'invention renferment en outre avantageusement une ou plusieurs substances actives, en particulier, une ou plusieurs vitamines, éventuellement un ou plusieurs sels minéraux, le zinc seul ou en combinaison, le calcium, le phosphore, le cuivre, le manganèse, le chrome, le fer, plus spécialement sous forme de sels ferreux avantageusement l'oxalate, éléments minéraux sous forme de sels, complexes ou autres dérivés, en particulier des sele de calcium.

Des vitamines appropriées sont constituées notamment par les vitamines A, B1, B2, B6, B12, PP, C, D2, E et I, selon les normes usuelles.

Les produits diététiques renferment en outre des additifs tels que correcteurs de goûts, par exemple des sucres ou des agents édulcorants, ou des acides arômatisants, des épices, des arômes, des parfums et excipients habituels.

Les formes de présentantion habituelles pour ces types de produits, telles que granulés, comprimés, pilules, ampoules, sirops, bonbons, pâtes à mâcher ou toute autre forme appropriée d'administration quelle qu'elle soit, par voie orale, injectable, parentérale, rectale ou autres, sont utilisées; en particulier pour les produits à usage pharmaceutique, les voies parentérales, injectables n'étant entreprises qu'en dernier lieu et en solution stérile et neutre sous la surveillance médicale usuelle.

Les granulés sont préférés en raison de leur facilité d'absorption. Ces granules sont préparés par des techniques usuelles, par exemple, à l'aide de cylindres à granulés ou par atomisation, pelletisation ou calandrage. Les granulés sont avantageusement à des dimensions de particules d'environ 0,5 à 5 mm et de préférence de 1 à 3 mm.

On peut faciliter la consommation des granulés par différents revêtements comme, par exemple, la dragéification de l'enveloppe. On peut utiliser pour ce faire, toutes les techniques usuelles et les substances autorisées par la législation sur les produits alimentaires. Les granulés peuvent contenir, en outre, des liants, des désagrégeants, des lubrifiants.

La dose individuelle quotidienne d'un produit diététique à usage humain, administée avantageusement en deux ou trois fois à un adulte, contient de 50 à 96g de protéines d'invertébrés marins, de préférence de l'ordre de 70 à 80g de protéines, le taux de 1g de protéines par kg de poids par jour étant généralement adopté, et éventuellement de 15 à 30 mg de fer, de préférence de l'ordre de 20 à 25 mg.

Ils contiennent en outre avantageusement de 2 à 4 g, de préférence de l'ordre de 3 g de pectine, des vitamines éventuellement aux quantités usuelles.

Dans le cas où l'on dispose de produits à usage pharmaceutique, renfermant des vitamines, on met en oeuvre ces dernières à raison d'environ 1500 à 1900 unités nationales de vitamines A, toujours pour un adulte. Pour les adolescents, les enfants au-dessus ou au-dessous de 2 ans, les doses de chaque vitamine seront modulées selon les indications du Codex ou directives du Ministère de la Santé de chaque pays concerné par l'invention. Les éventuelles contre-indications comme par exemple la vitamine A pour les nourrissons devront être respectées.

Cependant les vitamines suivantes pourront éventuellement être utilisées avantageusement selon les doses pour adultes indiquées ci-après:

Vitamine B1   :   de 0,2 à 0,6 mg
Vitamine B2   :   de 0,5 à 1 mg
Vitamine B12 :   de 1,5 à 2,5 µg
Vitamine C    :   de 30 à 50 mg
Vitamine E    :   de 30 à 50 mg

Les sels de calcium éventuellement mis en oeuvre dans ces produits, seront présents dans des proportions permettant d'administrer des doses quotidiennes de ces dernières de l'ordre de 200 à 450 mg, de préférence de 300 à 350 mg.

Une action de grande efficacité vis-à-vis des tissus cutanés a également été mise en évidence plus spécialement à base de protéines, éventuellement des acides aminés et éventuellement des peptides et éventuellement des hormones et éventuellement des oligo-éléments. On notera que l'emploi des vitamines autres que naturelles sera exclu.

L'utilisation des produits de l'invention en cosmétologie entre donc également dans le cadre de l'invention.

Dans cette application, on a recours à des formes appropriées pour l'usage externe et les applications locales, telles que de préférence crèmes, pommades, huiles, gels, laits, mascaras pour cils, masques de beauté ou encore shampooings.

Les produits de l'invention, avantageusement les protéines, et éventuellement les peptides et éventuellement les acides aminés (collagène, éventuellement avec des hormones, sont utilisés à raison de 1 à 20 parties en poids par rapport aux autres constituants, excipients et éventuellement substances actives mises en oeuvre.

L'un des constituants peut être le zinc. Les crèmes protéiniques pour la peau contenant du zinc doivent avoir une souplesse et une onctuosité permettant une application facile et un pH proche de celui du tissu cutané, compris entre 5,5 et 7. L'oxyde de zinc ZnO 25 g soit 25%. L'oxyde de zinc est préparé par calcination du zinc de façon usuelle àdes doses variant de 5 à 30% en ce qui concerne les poudres de beauté ou encore le stéarate de zinc $(C_{18}H_{36}O_2)_2Zn$ se préparant aussi couramment et employé à des doses de 5 à 10%, enfin l'undécylénate de zinc $((CH_3(CH_2)_9COO)_2Zn$ qui adhère bien à la peau.

L'application des produits de l'invention exerce un effet favorable sur la regénérescence des tissus et sur leur équilibre métabolique.

D'une manière avantageuse, on relève une tolèrance absolue des produits de l'invention utilisés aussi bien en diététique qu'en cosmétologie après différents tests sur différents sujets.

Pour extraire les composants désirés des animaux marins considérés, on a recours aux techniques habituelles appropriées à chaque type de composant.

Les protéines seront plus spécialement extraites sous forme de précipités obtenus par défécation de préférence trichloracètique ou à l'aide d'une solution de sulfate d'ammonium ou de sodium à 50% ou autre sel neutre, solvant organique ou tout moyen connu pour l'extraction des protéines.

Pour la récupération des protéines, on procède selon les techniques usuelles, par exemple par filtration, ultrafiltration, chromatographies et analogues. On peut également procèder par électrophorèses avec support ou non de l'électronique. La solution protéique peut être dosée par la méthode KJELDAHL sachant que statistiquement les protéines contiennent 16% d'azote. Par recoupement en laboratoire, on transforme l'azote en sulfate d'ammonium et on dose ensuite par acidimètrie après distillation.

Exemple de fabrication

Les étoiles de mer collectées sont congélées à −20°C ou maintenues dans l'eau de mer à salinité et à température ménagère pour qu'on puisse procèder à l'extraction des glandes (congélation dans les trois heures maxima de la prise).

Les glandes sont nettoyées soigneusement puis broyées par pilon ou mixées, puis centrifugées. Elles sont alors mélangées à de l'eau bidistillée refroidie avec du sulfate d'ammonium à 50%, les protéines brutes peuvent alors être précipitées par thermocoagulation s'il s'agit d'albumineuses ou pour protéines thermostables on peut encore faire une hydrolyse acide avec, par exemple, de l'acide chlorhydrique HCl 6N chauffé à 105°C pendant 24 h. L'acide est évaporé. Lavage et séchage.

Les protéines peuvent être identifiées en acides aminées, par exemple par chromatographie sur colonne échangeuse d'ions qui permet une analyse qualitative et quantitative. L'hydrolysat traverse la colonne à résine sulfonée puis est éluée par une solution tampon de citrate de sodium. On utilise deux colonnes: la première tamponnée à pH 5,3 sépare les acides aminés basiques; la seconde à pH 3,25 ou 4,25 sépare les autres acides aminés. L'éluat est mélangé à une solution de ninhydrine puis chauffé pendant 20 minutes. L'absorption des produits de la réaction avec la ninhydrine est mesurée dans un spectrophotomètre ce qui donne des pics reconnaissables.

Pour purifier les protéines on peut opérer par fractionnements avec filtration sur gel de silice ou électropforèse selon TISELIUS ou encore par ultracentrifugation par la méthode SVEDBERG...

L'hydrolysat s'opère de 24 h à 48 h maximum.

Les protéines thermostables pourront être amalgamées aux différents produits de l'invention ayant tous la même texture puis upérisées.

Les protéines thermolabiles, particulièrement les enzymes amalgamées aux produits de l'invention de préférence seront tyndallisées ou lyophilisées selon leur fragilité à la chaleur ou au froid, ou encore stérilisées par filtration.

Obtention de poudre acetonique

Les glandes ou tissus broyés et mixés par exemple 100 g, sont dissous dans 2 l de 0.1 M de $Na_2CO_3$ à pH 8.5 contenant 0.7M de NaCl. La mixture est agitée avantageusement pendant quatre heures à 4°C et centrifugée à 10 000 g (rotations par minute) pendant deux heures. Le supernatant est décanté et un solide par exemple $(NH_4)_2SO_4$ est ajouté jusqu'à ce que la saturation soit achevée. La matière est centrifugée de préférence à 33 000 g pendant 1 heure et le précipité obtenu est mis de côté au froid à −20°C jusqu'á utilisation.

Obtention des sels ferreux

Fe est libéré de ses complexes protéiques par hydrolyse pendant par exemple trois heures avec du HCl à pH 4,5. Après minéralisation par voie humide et après avoir réduit au préalable les ions ferriques $Fe^{3+}$ en ions ferreux $Fe^{2+}$ au moyen de l'hydroquinone par exemple, le fer sera dosé par colorimètrie de préférence en utilisant la méthode de l'ortho-phénantroline à pH 3,5 ou par la méthode des complexons III avec le réactif de PATTON et READER. L'oxalate de fer peut être dosé, par une solution de permanganate de potassium à 0,1N de préférence, sur filtre.

Le fer sera donc avantageusement extrait par hydrolyse du matériau de départ ou tout autre moyen couramment utilisé dans ce type de technique.

Exemple 1

Produit utilisable en diététique comme produit reconstituant

Les ingrédients suivants, en poudre sont mélangés selon les proportions respectives indiquées:

|  | Parties en poids |
|---|---|
| Protéines d'étoiles de mer | 30 |
| Pectine sèche de pomme | 1 |
| Sucre de betterave | 2 |

A partir de cette composition, on forme des granulés de 0,5 à 0,06 cg de 1,5 mm de long environ et de 1 mm de diamètre environ, par atomisation avec de l'eau dans un sèchoir tourbillonnaire à une température de l'ordre de 60°C environ puis passage dans une machine à granulés à cylindres. Les granulés sont séchés et tamisés une fois obtenus (ouvertures des mailles de 0,8 à 0,9 mm environ).

Selon une variante, on forme des dragées, en recouvrant des granulés avec un revêtement comme suit:

Sur des granulés obtenus à partir d'une composition formée de 30 parties en poids de protéines d'étoiles de mer, une partie en poids de pectine, deux parties en poids de sucre et 0,5 partie en poids de substances reconstituantes, on pulvérise, en couches fluidisées, une solution aqueuse sucrée renfermant de l'acide citrique. Ces dragées obtenues sont ensuite séchées à l'étuve.

6

On dispose ainsi de produits énergétiques, digestes, qui, par un apport adapté en éléments précieux pour l'organisme, permettent d'augmenter sont potentiel.

Ces produits sont particulièrement appropriés dans les cas d'asthénies et d'anémies, de rachitisme, de régénérescence et, d'une manière générale, de grande fatigue de l'organisme ou dans les cas de croissance.

Chez différents sujets ayant utilisé régulièrement un produit selon l'invention à raison de trois fois par jour, on a observé, dès la troisième semaine une amélioration nette de la forme physique.

Exemple 2

Produit reconstituant utilisable en diététique animale se présentant sous forme d'un bloc à lécher.

On prépare un bloc à lécher, de 2 à 3 kg selon l'animal à traiter, à l'aide de 1 à 45% de protéines d'invertébrés marins, de peptides et/ou d'acides aminés, de 1 à 5% d'éléments minéraux dont avantageusement 1% en fer, avantageusement 1 à 5% de vitamines.

D'une façon générale, on peut compter 1 g de protéines par kilo de poids de l'animal à traiter en besoin par jour et maximum 1 g à 1,2 g d'oxalate de fer pour une bête de 100 kg.

Dans cette variante, on ajoute 1 à 20% de glucides et éventuellement des lipides avantageusement d'invertébrés marins de préférence des étoiles de mer, 1 à 5% de pectine et 1 à 20% de poudre de lait séche, par rapport au poids total du bloc.

Le mélange de ces différents ingrédients est compressé avantageusement sous une pression de 180 kg/cm² pendant une à deux minutes. Les blocs à lécher sont d'un emploi facile mais on peut également utiliser les produits de l'invention destinés aux animaux sous toute autre forme appropriée, en leur ajoutant éventuellement des correcteurs de goût et en les mélangeant à la nourriture.

Exemple 3

Produits utilisables en cosmétologie—*Masque de beauté*:

On prépare un masque de beauté à l'aide des ingrédients:

1 à 70% en poids d'albumine,

1 à 70% en poids de produit astringent tel que la pulpe de concombre,

1 à 20% en poids de protéines (et/ou peptides et/ou acides aminés), accompagnés éventuellement d'hormones selon l'invention.

Après l'application, le masque est aisément enlevé avec de l'eau de rose.

Dans une variante, on utilise de l'argile, une composition lipidique telle que de l'huile d'olives, du citron et des protéines (et/ou peptides et/ou acides aminés), avec éventuellement des hormones selon l'invention.

Mascara pour cils

Les protéines et autres composés de l'invention sont mélangés à de l'huile de ricin.

Exemple 4

Obtention de protéines (thermostables avantageusement) d'étoiles de mer-

On soumet 100 kg d'étoiles de mer à un traitement comprenant leur lavage, séchage et broyage.

La poudre obtenue est soumise à un traitement d'upérisation et non de stérilisation dans des conditions permettant de détruire les parasites de l'étoile de mer tout en conservant les substances actives recherchées.

On procède aux opérations d'extraction selon les processus classiques par exemple à l'aide de sulfate de sodium.

**Revendications**

1. Produits pour usage pharmaceutique contre le rachitisme, l'anèmie, la fatigue, pour favoriser que la régénérescence et la croissance des organismes humains ou animaux, caractérisés en ce qu'ils renferment au moins l'élément zinc, à propriétés reconstituantes et revitalisantes, obtenu à partir des éléments physiologiques d'animaux marins invertébrés.

2. Produits pour usage pharmaceutique selon la revendication 1, caractérisés en ce que l'élément renfermé est l'élément minéral zinc, seul on en combinaison avec le calcium, le phosphore, le cuivre, le manganèse, le chrome ou le fer, plus spécialement sous form de sels ferreux, avantageusement l'oxalate, éléments mineraux sous forme de sels, complexes ou autres dérivés.

3. Produits pour usage pharmaceutique selon les revendications 1 à 2, caractérisés en ce que les éléments constituants obtenus à partir des éléments physiologiques sont des acides aminés, des peptides, des protéines, des enzymes, des hormones, des vitamines, des éléments minéraux, en particulier.

4. Produits pour usage pharmaceutique selon les revendications 1 à 3, caractérisés en ce que les éléments constituants sont extraits de différents éléments physiologiques tels que tissus, foie, estomac, gonades, sperme, ovaires, follicules, oeufs, glandes, secrétions, membranes et liquides cellulaires, organes, de préférence.

5. Produits pour usage pharmaceutique selon les revendications 1 à 4, caractérisés en ce qu'ils sont obtenus à partir de broyats.

6. Produits pour usage pharmaceutique selon les revendications 1 à 5, caractérisés en ce que ce sont des extraits.

7. Produits à propriétés reconstituantes et revitalisantes obtenus à partir des produits extraits, broyats, extraits obtenus à partir des éléments physiologiques des animaux marins invertébrés, caractérisés en ce qu'ils renferment, en outre une substance active appelée pectine, de préférence de la pectine sèche de pommes.

8. Produits selon la revendication 7, caractérisés en ce que les composants physiologiques sont des acides aminés ou des peptides ou des protéines ou des enzymes ou l'élément minéral zinc, seul on en combinaison avec le calcium, le phosphore, le cuivre, le manganèse, le chrome ou le fer, plus spécialement sous forme de sels ferreux, avantageusement l'oxalate, éléments minéraux sous forme de sels, complexes ou autres dérivés.

9. Produits selon la revendication 7, caractérisés en ce que les composants physiologiques sont des acides aminés et des peptides et des protéines et des enzymes et l'éléments minéral zinc, seul on en combinaison avec le calcium, le phosphore, le cuivre, le manganèse, le chrome ou le fer, plus spécialement sous forme de sels ferreux, avantageusement l'oxalate, éléments minéraux sous forme de sels, complexes ou autres dérivés.

10. Produits selon les revendications 7 à 9, caractérisés en ce que les composants physiologiques d'animaux marins invertébrés sont extraits de différents éléments tels que tissus, foie, estomac, glandes, organes, sécrétions, oeufs, sperme, gonades, ovaires, follicules, membranes et liquides cellulaires, de préférence.

11. Produits selon les revendications 7 à 10, caractérisés en ce qu'ils renferment 1 à 45% de protéines et/ou de peptides et/ou d'acides aminés, 1 à 20% de glucides et/ou lipides, des hormones, des vitamines selon les normes usuelles, une quantité efficace d'au moins l'un des composés des produits évoqués.

12. Produits selon les revendications 7 à 11, caractérisés en ce qu'ils renferment en outre des additifs tels que des correcteurs de goût ou des acides arômatisants, des épices, des arômes, des parfums et excipients habituels.

13. Produits à usage diététique humain, caractérisés en ce qu'ils renferment avantageusement pour un adulte une dose quotidienne de 50 à 96 g de protéines d'invertébrés marins, de préférence de 70 à 80 g, et/ou 15 à 30 mg de fer, de préfécence de 20 à 25 mg, et contiennent en outre de 2 à 4 g, de préférence de 3 g de pectine, des vitamines éventuellement aux quantités usuelles et des sels de calcium éventuellement de 200 à 450 mg, de préférence de 300 à 350 mg.

14. Produits à usage diététique animal, caractérisés en ce qu'ils se présentant avantageusement sous forme d'un bloc à lécher de 2 à 3 kg selon l'animal à traiter et renferment de préférence de 1 à 45% de protéines d'invertébrés marins et/ou de peptides et/ou d'acides aminés, de 1 à 5% d'éléments minéraux dont avantageusement 1% en fer, avantageusement 1 à 5% de vitamines, 1 à 20% de glucides et/ou lipides avantageusement d'invertébrés marins, de préférence des étoiles de mer, 1 à 5% de pectine et 1 à 20% de poudre de ladite sèche, par rapport au poids total du bloc, le mélange de ces différents ingrédients étant avantageusement comprimé sous une pression de 180 kg/cm$^2$ pendant une à deux minutes.

15. Utilisation en diététique humaine des produits définis selon les revendications 1 à 13.

16. Utilisation en diététique animale des produits définis selon les revendications 7 à 12, et 14.

17. Utilisation en diététique selon les revendications 13 et 14, sous des formes telles que granulés, sirops, pilules, ampoules, comprimés, bonbons, pâtes à mâcher ou toute autre forme pour l'administration, de préférence par voie orale, injectable, parentérale, rectale.

18. Utilisation en cosmétique des produits définis selon les revendications 7 à 12, caractérisés en ce qu'ils se présentent sous des formes appropriées pour l'usage externe et les applications locales telles que, de préférence, crèmes, pommades, huiles, gels, laits, mascaras pour cils, masques de beauté ou encore shampooings.

## Patentansprüche

1. Produkte für den Apothekegebrauch gegen der Rachitis, Blutarmut, um mit dem Wiederaufleben und der Menschen oder Tierenorganismene Wachstum zuwirken, dadurch gekennzeichnet, dass sie in sich wenigstens Zinkelement im wesentlichen wiederaufbauende und revitalisierende Eigenschaften besitzten, die aus ihren den Wirbelloseseetieren physiologischen Elementen erhalten.

2. Produkte für den Apothekegebrauch, gemäss Patentanspruch 1, dadurch gekennzeichnet, dass der Bestandteil enthaltet Mineralzinkelement ist, also ganz allein oder in Verbindung mit dem Calcium, Phosphor, Kupfer, Mangan, Chrom oder dem Eisen insbesondere in Form von Ferrosalzen, vorteihafterweise von Oxalsalzen, Mineralelementen in Form von Salzen, Komplexen oder anderen Derivaten bestehen.

3. Produkte für den Apothekegebrauch, gemäss Patentansprüche 1 bis 2, dadurch gekennzeichnet, dass die Bestandteil bildenden Verbindungen, die aus den Wirbellose Seetieren physiologischen Elementen erhalten, Aminosäuren, Peptiden, Proteinen, Enzymen, Hormonen, Vitaminen, insbesondere Mineralelementen sind.

4. Produkte für den Apothekegebrauch, gemäss Patentansprüche 1 bis 3, dadurch gekennzeichnet, dass die Bestandteil bildenden Verbindungen aus manigfahren physiologischen Elementen erhalten als solche Geweben, Leber, Magen, Gonaden, Sperma, Ovaria, Follikeln, Eiern, Drüse, Absonderungen, Membranen und Zellenliquida, Organen inbesonders sind.

5. Produkte für den Apothekegebrauch, gemäss Patentansprüche 1 bis 4, dadurch gekennzeichnet, dass sie von den Brecheren erhalten.

6. Produkte für den Apothekegebrauch, gemäss Patentansprüche 1 bis 5, dadurch gekennzeichnet, dass die Extraction sind.

7. Produkte, die im wesentlichen wiederaubauende und revitalisierende Eigenschaften besitzen, die aus wirbellosen Meerestieren von Extrakten, Brecheren an oder Produkte, die von physiologischen Elementen den wirbellosen Meerestieren an geworden werden können, dadurch gekennzeichnet, dass sie ausserdem eine aktive Pektinsubstanz, vorzusweise Apfeltrockenpektin, enthalten.

8. Produkte gemäss Patentansprüch 7, dadurch gekennzeichnet, dass die physiologischen Bestandteil Aminosäuren sind oder Peptide Oder Proteine oder Enzyme oder der Mineralzink, allein oder in Verbindung, mit dem Calcium, Phosphor, Kupfer, Mangan, Chrom oder dem Eisen, insbesondere in Form von Ferrosalzen, vorteilhafterweise von das Oxalsalzen, Mineralelementen in Form von Salzen, Komplexen oder anderen Derivaten bestehen.

9. Produkte gemäss Patentanspruch 7, dadurch gekennzeichnet, dass die physiologischen Bestandteil Aminosäuren sind und Peptide und Proteine und Enzyme und der Mineralelementzink, allein oder in Verbindung mit dem Calcium, Phosphor, Kupfer, Mangan, Chrom oder mit dem Eisen, insbesondere in Form von Ferrosalzen, vorteilhafterweise von das Oxalsalzen, Mineralelementen in Form von Salzen, Komplexen oder anderen Derivaten bestehen.

10. Produkte gemäss Patentansprüche 7 bis 9, dadurch gekennzeichnet, dass die Bestandteil bildenden Verbindungen, die aus den wirbellose Seetieren physiologischen Elementen geextrahiert sind von wie Geweben, Leber, Magen, Drüse, Organen, Absonderungen, Eiern, Sperma, Gonaden, Ovaria, Follikeln, Membranen und Zellenliquida, insbesondere.

11. Produkte gemäss Patentansprüche 7 bis 10, dadurch gekennzeichnet, dass sie 1 bis 45% Proteine und vorteilhaft Peptide oder vorteilhaft Aminosäuren, 1 bis 20% Kohlehydrate oder vorteilhaft Lipide, Hormone, Vitamine, sowie die üblichen Bindemittel, eine wirkende Quantität viel wenigstens als eine Bestandteil den herrüfteten Produkten herauf, enthalten.

12. Produkte gemäss Patentsprüche 7 bis 11, dadurch gekennzeichnet, dass sie auch Zusätze wie schmachskompensatoren oder aromatisierende Säuren, Gewürze, Aroma, Wohlgerüche und die üblichen Bindemittel.

13. Produkte für menschlichen diätetischen Gebrauch, dadurch gekennzeichnet, dass sie vorzugsweise bei einem Erwachsenen in Tagesdosen verarbreicht werden, die vorteilhaft 50 bis 96 g wirbellose Meerestierenproteine, vorteilhaft 70 bis 80 g, und vorzugsweise 15 bis 30 mg Eisen, vorteilhaft 20 bis 25 mg, und 2 bis 4 g vorzugsweise 3 g Pektin, Vitamine etwalgen Falls sowie die üblichen Bindemittel und Calciumsalze möglichen Falls 200 bis 450 mg, vorzugsweise 300 bis 350 mg, enthalten.

14. Produkte für tierischen diätetischen Gebrauch, dadurch gekennzeichnet, dass sie vorteilhaft in Form eines abzuleckenden Blockes von 2 bis 3 kg, je nach zu behandelndem Tier, erhältlich sind und vorteilhaft 1 bis 45% wirbellose Meerestierenproteine, vorzubsweise Peptide, vorzugsweise Aminosäuen, 1 bis 5% Mineralelementen, davon vorzugsweise 1% Eisen, vorteilhaft 1 bis 5% Vitamine, 1 bis 20% Kohlehydrate und/oder liquide, die vorzugsweise aus wirbellosestieren gewonnen werden, vorteilhaft aus Seesternen, 1 bis 5% Pektin und 1 bis 20% Trocknetesmilchpulver, in Rücksicht auf gänzlichen Gewicht des Blockes, die mischung dieser verschiedenen Zutaten vorteilhaft unter einem Druck von 180 kg/cm$^2$ ein bis Zwei Minuten lang komprimiert sind.

15. Nutzbarmachung in menschlichen diätetischen Gebrauch der Produkte, solche gemäss die Patentansprüche 1 bis 13 enthalten.

16. Nutzbarmachung in tierischen diätetischen Gebrauch der Produkte, solche gemäss die Patentansprüche 7 bis 12, und 14 enthalten.

17. Nutzbarmachung in Diätetick gemäss Patentansprüche 13 und 14, dadurch gekennzeichnet, dass sie in Form von Grundulat, Sirup, Pillen, Bläschen, Tabletten, Bonbons, Kaupasten oder in jeder anderen Form zu Einnehmen vorteilhaft durch den Mund, durch Einspritzen, durch Parenterale, durch Rektale, erhaltet sind.

18. Nutzbarmachung in Kosmetick gemäss Patentansprüche 7 bis 12, dadurch gekennzeichnet, dass sie in geeigneten Form auf den Aussengebrauch und die Ortsauflegen wie vorteilhaft Kreme, Pomade, Öle, Geele, Milche, Schminke für Augenwinper, Schönheitmaske, oder auch Kopfwäsche, erhalten sind.

## Claims

1. Products for pharmaceutical use against rickets, anaemia, fatigue, to favorise regeneration and the growth of human or animal organisms, characterised in that it includes the element zinc, which has reconstitutive and revitalising properties obtained from the physiological elements of invertebrate marine animals.

2. Products for pharmaceutical use according to Claim 1, characterised in that the element contained is the mineral element zinc, either alone or in combination with calcium, phosphorus, copper, magnanese, chromium or iron, more especially in the form of iron salts, advantageously oxalate, mineral elements in the form of salts, complexes or other derivatives.

3. Products for pharmaceutical use according to Claim 1 or 2 characterised in that the constituent elements obtained from physiological elements are amino acids, peptides, proteins, enzymes, hormones, vitamins, and mineral elements in particular.

4. Products for pharmaceutical use according to Claims 1 to 3, characterised in that the constituent elements are extracted from the various physiological elements, such as tissues, liver, stomach, gonads, sperm, ovaries, follicles, eggs, glands, secretions, membranes and cellular liquids, organs, in preference.

5. Products for pharmaceutical use according to Claims 1 to 4 characterised in that they are obtained from grindings.

6. Products for pharmaceutical use according to Claims 1 to 5, characterised in that they are extracts.

7. Products with reconstituant and revitalising properties obtained from extract products, grindings, extracts obtained from physiological elements of invertebrae marine animals, characterised in that they contain, in an active substance called pectine, preferably the pectine of dried apples.

8. Products according to Claim 7, characterised in that the physiolocial components are amino acids or peptides or proteins or enzymes or the mineral element zinc, alone or in combination with calcium, phosphorous, copper, manganese, chromium or orin, more especially in the form of iron salts, advantageously oxalate, mineral elements in the form of salts, complexes or other derivatives.

9. Products according to Claim 7, characterised in that the physiological components are amino acids and peptides and proteins and enzymes and the mineral element zinc, alone or in combination with copper, manganese, chromium, iron, more especially in the form of iron salts, advantageously oxalate, mineral elements in the form of salts, complexes or other derivatives.

10. Products according to Claims 7 to 9 characterised in that the physiological components of invertebrate marine animals are extracted from various elements such as liver, stomach, glands, tissues, organs, secretions, eggs, sperm, gonads, ovaries, follicles, membranes and cellular liquids, in preference.

11. Products according to Claims 7 to 10, characterised in that they contain 1 to 5% proteins and/or peptides and/or amino acids, 1 to 20% glucides and or/lipides, hormones, vitamines per the usual norms, an efficient quantity of at least one of the compounds of the products mentioned.

12. Products according to Claims 7 to 11, characterised in that they contain, in addition, additives such as taste enhancers or aromatic acids, spices, aromas, fragrances and the usual excipients.

13. Products for huamn dietary use, characterised in that they contain advantageously for an adult a daily dose of 50 to 96 grams of invertebrate marine proteins, preferably from 70 to 80 grams and/or 15 to 30 mgs of iron, though preferably from 20 to 25 mgs, and also comprise 2 to 4 grams, though preferably 3 grams of pectin, possibly vitamins in the usual quantities and normally 200 to 400 mgs. of calcium salts, though preferably 300 to 350 mgs.

14. Products for animal dietary use, characterised in that they are advantageously presented in the form of a two or three kilo lump, depending on the animal concerned, said lumps to be licked, with a reinforcement of preferably 1 to 45% invertebrate marine proteins, and/or peptides and or amino acids, from 1 to 5% mineral elements, of which advantageously 1 to 5% vitamins, 1 to 20% glucides and/or advantageously lipides of marine invertebrates, preferably starfish, 1 to 5% pectins and 1 to 20% dried milk powder relative to the weight of the whole lump, the mixture made up of these various ingredients being advantageously compressed at a pressure of 180 kg/cm² for two minutes.

15. The use for human dietetics of products defined according to Claims 1 to 13.

16. The use for animal dietetics of products defined according to Claims 7 to 12 and 14.

17. Use in dietetics according to Claims 13 and 14, in the form of granulates, syrup, pills, phials, tablets, sweets, chewing gum or any other form of preferably the oral type of administering these but also by injection, parenteral or rectal means.

18. The use in cosmetics, of the products defined according to Claims 7 to 12, characterised in their presentation in the appropriate forms for external use and local applications, such as, preferably, creams, ointments, oils, gels, milks, eyelash mascaras, beauty face packs or even shampoos.